# EUROPEAN PATENT SPECIFICATION

(11) **EP 2 248 522 B1**
(45) Date of publication and mention of the grant of the patent: **03.05.2017**
(21) Application number: 09708715.9
(22) Date of filing: 05.02.2009
(51) Int. Cl.: A61K 31/436, A61P 37/06, A61P 17/00, A61K 9/00, A61K 9/14, A61K 47/26, A61K 47/36, A61K 47/38, A61K 47/12, A61K 47/20

(54) **PREPARATION OF AN IMMUNOSUPPRESSIVE MACROLIDE POWDER FOR ORAL SUSPENSION**
HERSTELLUNG EINES IMMUNSUPPRESSIVEN MAKROLIDPULVERS FÜR ORALE SUSPENSIONEN
PRÉPARATION D'UN POUDRE POUR SUSPENSION ORALE D'UN MACROLIDE IMMUNOSUPPRESSEUR

(30) Priority: 05.02.2008 CL 3742008
(43) Date of publication of application: 10.11.2010
(73) Proprietor: Igloo Zone Chile S.a., Santiago de Chile (CL); Laboratorios Synthesis S.A.S., Bogota D.C. (CO)
(72) Inventor: IVÁN RAMIREZ MONETTA, Rodrigo, Santiago de Chile (CL)
(74) Representative: Hart, Deborah Mary
(86) International application number: PCT/IB2009/050455
(87) International publication number: WO 2009/098649

(56) References cited:
- EP-A1- 0 484 936
- WO-A1-2006/083130
- WO-A1-2008/013416
- US-A1- 2003 235 614
- US-A1- 2006 135 548
- A ELEFANTE ET AL: "Long-term stability of a patient-convenient 1?mg/ml suspension of tacrolimus for accurate maintenance of stable therapeutic levels", BONE MARROW TRANSPLANTATION, vol. 37, no. 8, 1 April 2006 (2006-04-01), pages 781-784, XP055045615, ISSN: 0268-3369, DOI: 10.1038/sj.bmt.1705320
- Paddock Laboratories Inc.: "ORA-SWEET", , 2010, XP002688020, Retrieved from the Internet: URL:http://www.stobec.com/documents/data/8 196.pdf [retrieved on 2012-11-27]
- JACOBSON, P.A ET AL.: 'Stability of tacrolimus in an extemporaneously compounded oral liquid' AM. J. HEALTH-SYST PHARM vol. 54, no. 15, 1997, pages 178 - 180, XP008139588
- PLOSKER, G.L. ET AL.: 'Tacrolimus. A further update of its pharmacology and therapeutic use in the management of organ transplantation' DRUGS vol. 59, no. 2, 2000, pages 323 - 389, PAG, XP008139590
- GONZALEZ DE OLANO, D. ET AL.: 'Tacrolimus como tratamiento of the dermatitis at6pica:estudio piloto observacional in the práctica clinica.' ALERGOL. INMUNOL. CLIN. vol. 18, 2003, pages 269 - 273, XP008139976

## Description

### FIELD OF THE APPLICATION

The present invention describes pharmaceutical compositions that comprise a tacrolimus powder for oral suspension that exhibits great stability as a powder for suspension and also, once prepared as the extemporaneous suspension, without the formation of cake-like clusters, having a satisfactory flavour and a pleasant aroma. The disclosure also describes the method for preparing the pharmaceutical compositions, same being a dry method that comprises mixing tacrolimus and pre-sieved pharmaceutically acceptable carriers for a suitable length of time. Also disclosed herein is the use of the pharmaceutical compositions for treating and preventing rejection of transplanted organs and atopic dermatitis

### DESCRIPTION OF THE PRIOR ART

Tacrolimus is an immunosuppresive pharmaceutical, widely administered orally and intravenously for the prevention and treatment of the rejection of transplanted organs, mainly the liver and kidneys (Plosker GL, Foster RH. Tacrolimus: a further update of its pharmacology and therapeutic use in the management of organ transplantation. Drugs. 2000, 59(2):323-89). It also used topically for the treatment of atopical dermatitis (González de Olano D; Roan Roan J, de la Hoz Caballer D, Amaruch Garcia N, Moral Jiménez S, Murie A, Sánchez Cano M. Tacrolimus como tratamiento de la dermatitis atópica: estudio piloto observacional en la práctica clínica (Tacrolimus as a treatment for atopical dermatitis: observational pilot study in clinical practice). Alergol. Inmunol. Clin. 2003, 18: 269-273).

Tacrolimus performs its therapeutical effect after combining with the protein FKBP-12 and forming the complex tacrolimus-FKBP-12-calcium-calmodulin-calcineurin. This complex inhibits the phosphatase activity of calcineurin, thus avoiding the dephosphorylation and displacement of the nuclear factor of the activated T lymphocytes (NF-AT), nuclear component that intervenes in the transcription of the genes that form the cytokines, such as interleukin (IL-2, IL-3, IL-4, IL-5, TNF-α and GM-CSF) and the interferon-χ(IFN-χ). These cytokines participate in the first steps of the activation of the T lymphocytes, for which tacrolimus inhibits the activation of said cell mediators. It has also been shown that *in vitro,* tacrolimus decreases the release of the mediators of skin mast cells, basophils and eosinophils (Plosker GL, Foster RH. Tacrolimus: a further update of its pharmacology and therapeutic use in the management of organ transplantation. Drugs. 2000, 59 (2) :323-89) .

The absorption of tacrolimus after oral administration is variable, and low solubility and gastrointestinal motility have an impact. The maximum concentration (Cmax) in the blood stream is reached within 1-3 hours on average, when it is administered orally (0.3 mg/Kg/day) for patients that have received a liver transplant, the concentration in steady state is reached after 3 days in the majority of the cases. Bioavailability is low and very variable at 4-89% and is reduced with the presence of food. It is distributed in the majority of the tissues, presents a distribution rate of 0.85 to 1.94 L/Kg and a high combination to proteins (99%), mainly to albumin and to α1-glycoprotein, and it also binds with erythrocyte in the blood in a total plasma:blood rate of 20:1. It is metabolized through intestinal and hepatic cytochrome P450 (CYP3A4), whereby the main metabolites are 13-O-desmethyl and 15-O-desmethyl tacrolimus, neither of which have a measurable immunosuppresive effect. The elimination half-life varies between 12 and 19 hours. The main channel of elimination is through bile (more than 90%) and less than 1% is excreted in unaltered state through urine (Bartlomiejczyk I, Zochowska D, Sanko-Resmer J, Matuszewicz D, Paczek L. Therapeutic monitoring of tacrolimus concentrations in blood of renal and liver transplant recipients: comparison of microparticle enzyme immunoassay and enzyme multiplied immunoassay methods. Transplant. Proc. 2006, 38(1):94-96); Venkataramanan R, Swaminathan A, Prasad T, Jain A, Zuckerman S, Warty V, McMichael J, Lever J, Burckart G, Starzl T.Clinical pharmacokinetics of tacrolimus. Clin. Pharmacokinet. 1995, 29(6):404-30).

Tacrolimus is a macrolide with the global formula C₄₄H₆₉NO₁₂, P.M.804,02, mp 127-129° (anhydrous), C₄₄H₆₉NO₁₂xH2O, P.M.: 822,03 (monohydrate), produced by the bacteria *Streptomyces tsukubaensis.* Chemically it corresponds to [3S - [3R* [E (1S*, 3S*, 4S*)], 4S*, 5R*, 8S*, 9E, 12R*, 14R*, 15S*, 16R*, 18S*, 19S*, 26aR*]]-5, 6, 8, 11, 12, 13, 14, 15, 16, 17, 18, 19, 24, 25, 26, 26a - hexadecahydro - 5, 19-dihydroxy - 3 - [2 - (4-hydroxy-3-methoxy-cyclohexyl) -1-methylethenyl] - 14, 16-dimethoxy-4, 10, 12, 18 - tetramethyl - 8 - (2-propenyl)- 15, 19-epoxy - 3H-pyrido [2,1-c] [1,4] oxazacyclotricosine-1, 7, 20, 21 (4H, 23H) - tetraone, monohydrate. It is insoluble in water and soluble in methanol, ethanol, acetone, ethyl acetate, chloroform and diethyl ether (The Merk index-14 edition).

The commercial tacrolimus products that have been delevoped up to now are capsules, ointments and also come under the form of intravenous infusion, which present some problems regarding stability, bioavailability and are difficult to administer to the elderly and to children.

The physiochemical characteristics of tacrolimus, especially its low solubility in water and low wetting are convenient for the formulation of a pharmaceutical composition for oral administration that ensures good stability, gastrointestinal absorption and adequate bioavailability.

In order to resolve these inconveniences, different formulations have been developed, which in some cases can be incorporated in pharmaceutical forms for oral administration such as capsules, tablets and syrups. Herein we can find solid dispersions with added enteric polymers (WO2006052098), nanoparticles smaller than 2000 nm and added surface stabilizers (WO2006066063), preparations for oral administration that include the addition of surface agents and dissolvents such as ethanol and povidone (CN1919186-2007-02-28), solid dispersions with enteric coating (US2006287352), solid dispersions wherein the pharmaceutical has been dissolved and/or dispersed in a hydrophilic vehicle or miscible with water to form a dispersed solid or a solid solution at room temperature (WO 2005020994) and the formation of granules (WO2007091109), emulsions formulated with the addition of co-surfactants, surfactants and oils (WO2006062334).

In the case of the pharmaceutical formulations in solution, greater solubility of tacrolimus is achieved by adding surfactants, agents that reduce the superficial tension of a solution, allowing for the dissolution and emulsification of different substances that are insoluble in water. Generally, surfactants of vegetable or animal origin are used, or synthetic cationic, anionic or non-ionic compounds are used; however, the selection of an adequate surfactant to prepare a stable solution for tacrolimus is difficult. Because of its physiochemical characteristics, such as its hydrophilic-lipophilic balance (HLB) and its critical micelle concentration (CMC), it is preferable to use the castor oil polyethylene oxide (Korean patent N° 0177158, Chung Y, Cho H. Preparation of highly water soluble tacrolimus derivatives: poly(ethylene glycol) esters as potential prodrugs. Arch. Pharm. Res. 2004, 27(8):878-83). Castor oil, also known as ricin oil, can produce hypersensibility and/or gastrointestinal problems, the latter as a result of the irritating laxative effects which can even lead to hydrolytic disorders with hypokalemia. The above described adverse effects acquire greater relevance if tacrolimus is administered in pediatric patients or the elderly, when it is indicated to avoid the rejection of a transplanted kidney and even more given that among the adverse effects of tacrolimus we find nephrotoxicity and gastrointestinal problems, including abdominal pain and diarrhea.

Furthermore, in pharmaceutical compositions of powder for suspension, the main obstacle is their stability once they are reconstituted and during their period of effect. In the case of tacrolimus, it tends to precipitate after long periods of storage, its pharmaceutical stability may decay and with it the content of the active principle may decrease.

Jacobson et *al.* and Han et *al.* (Jacobson PA, Johnson CE, West NJ, Foster JA. Stability of tacrolimus in an extemporaneously compounded oral liquid. Am J Health Syst Pharm. 1997, 54(2):178-80; Han J, Beeton A, Long PF, Wong I, Tuleu C. Physical and microbiological stability of an extemporaneous tacrolimus suspension for paediatric use. J Clin Pharm Ther. 2006, 31(2):167-72) evaluated the physical and microbiological stability of a prepared liquid oral formulation suspending the content of tacrolimus capsules that are commercially available in ora-plus and common syrup. The authors postulate that the prepared suspension remains stable under storage conditions (24-26°C), given that at least 98% of the initial concentrations of tacrolimus determined by HPLC remained in the suspension throughout the observation period (0, 7, 15, 45 and 56 days), with no color or pH variations and without bacterial or fungal contamination. These parameters can be considered to determine the stability of a formulation as the one described in the current invention.

The preparation methods for the formulations that contain tacrolimus fundamentally consist in dissolving the active principle into organic dissolvants, hydrophilic polymers, adding surfactants or the formation of liposomes and subsequently submitting them to drying and conditioning treatments of the drug, which increment the fabrication time and cost. Therefore, it is necessary to have a formulation of tacrolimus for oral administration with adequate dissolution and stability qualities to ensure good bioavailability. At the same time, this formulation should be elaborated in a simple manner, with as little steps as possible, efficiently, low-cost and not requiring any special laboratory equipment.

### DETAILED DESCRIPTION

The present invention describes a procedure for the preparation of the pharmaceutical composition for oral suspension of Tacrolimus or a pharmaceutically acceptable salt, hydrate or solvate thereof said composition being a powder comprising Tacrolimus or a pharmaceutically acceptable salt, hydrate or solvate thereof, a colourant and a diluent or optionally other pharmaceutical excipients; the process consisting of sieving each of the components of the formulation, adding them to a mixer in order, starting with the diluent and leaving the tacrolimus and the colorant for last, mixing for 20 to 30 minutes, placing into tared containers and dose in vials.

The present invention describes pharmaceutical compositions that consist in a stable powder for oral suspension, that remains chemically stable once the extemporaneous suspension has been prepared, without the formation of cake-like clusters and lacking microbiological contamination and that furthermore has a good taste, a pleasant aroma and improved bioavailability compared to solid forms of oral administration.

The above mentioned properties are possible thanks to the incorporation of buffering substances that avoid the degradation of the active principle by providing the aqueous suspension with a pH of 3.5-4.5 once it is reconstituted and thanks to the addition of other pharmaceutically acceptable excipients such as thickening agents, preservatives, diluants, anti-adherents, sweeteners, colorants and flavors.

The pharmaceutically acceptable excipients are chosen from: citric acid and its pharmaceutically acceptable salts such as anhydrous sodium citrate and sodium citrate dihydrate as buffers (0.5 -10.0% in weight of the final formulation); guar gum, xanthan gum, tragacanth, carmellose, methylcellulose, ethylcellulose, propylcellulose, hydroxymethylcellulose, carboxymethylcellulose, aluminium silicate, magnesium silicate, polyvinyl alcohol, carbomer, gelatin, maltodextrine and polydextrose as thickening agents (0.5-10.0% in weight of the final formulation); sodium sorbate, potassium sorbate, sodium benzoate, phenylmercury acetate, phenylmercury nitrate, sodium propionate and thiomersal as preservatives (0.1-5.0% in weight of the final formulation); sorbitol, calcium phosphate, calcium sulfate, fructose, kaolin, magnesium carbonate, maltose, microcrystalline cellulose, and pregelatinized starch as diluants (10.0-95.0% in weight of the final formulation); colloidal silica dioxide, calcium stearate, magnesium oxide and talcum as an anti-adherent (0.1-5.0% in weight of the final formulation); sucralose, sucrose, aspartame, sodium saccharine, sodium cyclamate, fructose, maltose and sorbitol as sweeteners (0.1-5.0% in weight of the final formulation); tutti frutti essence, vanilla essence and menthol as flavors (0.1-5.0% in weight of the final formulation), FD and C yellow N° 6, FD and C red N° 40, betacarotene, and iron oxide as colorants (0.001-0.5% in weight of the final formulation).

The resulting formula disclosed herein is a powder that once reconstituted in water generates a fluid suspension, without the formation of cake-like clusters, easily resuspended after a slight agitation with a density of 1.05-1.11 g/mL and a pH of 3.5-4.5.

The majority of tacrolimus suspensions contain surfactants to stabilize the suspension. Surprisingly, the formulation disclosed herein achieves a stable suspension without incorporating surfactants, hereby avoiding hypersensibility and/or gastrointestinal problems that are typical for these types of substances, mainly seen in pediatric and geriatric patients. This behavior is in no way predictible for a technician who is well-versed in the subject matter.

Furthermore, the proposed pharmaceutical composition presents the advantage that it does not require the tacrolimus to be dissolved in organic dissolvants, nor the formulation of protective films, nanoparticles or granules that increase the cost of the formulation.

The preparation procedure described in the current invention, equally new and low-cost, consists in sieving each one of the components of the formulation, adding the different elements to the mixer, starting with the diluant and ending with the tacrolimus and the colorant, mixing appropriately (for 20-30 minutes), place into tared containers and dose in vials.

### Examples

Table 1 shows examples of pharmaceutical compositions of tacrolimus 1mg/mL as proposed in the current disclosure.

**Table 1. Pharmaceutical compositions.**

| | Example 1 | Example 2 | Example 3 | Example 4 |
|---|---|---|---|---|
| Tacrolimus | 0.3572 g | 0.3572 g | 0.3572 g | 0.3572 g |
| Anhydrous citric acid | 1.7857 g | 1.7857 g | 1.7857 g | 1.7857 g |
| Sodium citrate x 2 H₂O | 1.0714 g | 1.0714 g | 1.0714 g | 1.0714 g |
| FD and C yellow die N° 6 | 0.0036 g | 0.0040 g | 0.0032 g | 0.0032 g |
| Sucralose | 0.4286 g | 0.5328 g | - | 0.4853 g |
| Sodium cyclamate | - | - | 0.5618 g | - |
| Tutti Fruti essence | 1.1429 g | 1.1429 g | 1.1429 g | |
| Xhantan gum | 1.0000 g | 1.0000 g | 1.2000 g | |
| hydroxylpropylmet hylcellulose | - | 0.2000 g | - | 1.0000 g |
| Magnesium silicate | - | - | - | 0.2000 g |
| Colloidal silica dioxide | 0.3571 g | 0.3598 g | 0.3622 g | 0.3622 g |
| Potassium sorbate | 0.9000 g | 0.9500 g | 0.9300 g | 0.9300 g |
| Sodium benzoate | 0.5143 g | 0.6138 g | 0.6522 g | 0.6522 g |
| QS Sorbitol | 100.0000 g | 100.0000 g | 100.0000 g | 100.0000 g |

The method of manufacturing of the pharmaceutical composition of tacrolimus powders for suspension proposed in the current invention, consists in:
1. Sieving the components of the formulation through a N° 40 mesh.
2. Placing all the components in an adequatly sized mixer, initiating the proces with a selected diluant following the reverse order of appearance in the formula, ending with the tacrolimus and colorant. Mix during 20 - 30 minutes.
3. Place in tared containers with a double polyethylene bag.
4. Dose in adequately sized vials, preferably amber European hydrolytic type III, provide with safety cover.

The pharmaceutical composition of example 1 elaborated using the previously described method, once reconstituted, reserves its physiochemical and microbiological characteristics during the entire usage period of the suspension.

### Stability testing of the pharmaceutical composition (example 1)

7g of the tacrolimus powder 1mg/ml for suspension contained in amber European hydrolytic type III vials provided with a plastic safety cover, was reconstituted with 20 ml of purified water to obtain 25 ml of suspension and maintained at 25° C ± 2° C, 60% ± 5% R.H. The visual aspect, the pH, the tacrolimus content (titration) and the content of impurities were assessed 8, 15, 22 and 30 days after the suspension was prepared and were compared with the characteristics that it presented when the suspension was just reconstituted (initial condition).

The results of the 3 independently performed stability tests for the suspension reconstituted in water of the tacrolimus powder for suspension in agreement with example 1.

The results show that the suspensions maintained a fluid aspect, an orange color, a smell of fruit and a sweet taste throughout the entire assessment period. The pH remained within the optimal range of 3.5 - 4.5 and the observed fluctuation was less than 3.5%. The content of the active principal tacrolimus shows a slight decrease, this decrease being less than 5% at day 30. No chromatographic impurities were detected initially nor after 8 días, and were less than 2% at day 30, indicating that no degradation products appear of the active principle or the pharmaceutically acceptable excipients used in the formulation during the efficiency period of the reconstituted suspension.

Based on the physical and chemical stability data, the selected packaging components for the formulations described in the current invention are vials of amber hydrolitic type III provided with plastic safety covers and the storage conditions of a cool and dry place, at no more than 25° C and protected from direct sunlight.

## Claims

1. A procedure for the preparation of the pharmaceutical composition for oral suspension of Tacrolimus or a pharmaceutically acceptable salt, hydrate or solvate thereof said composition being a powder comprising Tacrolimus or a pharmaceutically acceptable salt, hydrate or solvate thereof, a colourant and a diluent or optionally other pharmaceutical excipients; the process consisting of sieving each of the components of the formulation, adding them to a mixer in order, starting with the diluent and leaving the tacrolimus and the colorant for last, mixing for 20 to 30 minutes, placing into tared containers and dose in vials.

## Patentansprüche

1. Verfahren zur Zubereitung einer pharmazeutischen Zusammensetzung für eine orale Suspension von Tacrolimus oder einem pharmazeutisch akzeptablen Salz, Hydrat oder Solvat davon, wobei die Zusammensetzung ein Pulver ist, umfassend Tacrolimus oder ein pharmazeutisch akzeptables Salz, Hydrat oder Solvat davon, einen Farbstoff und ein Verdünnungsmittel oder wahlweise weitere pharmazeutische Trägerstoffe; wobei das Verfahren im Sieben jeder der Komponenten der Formulierung, nacheinander Einbringen in einen Mixer, beginnend mit dem Verdünnungsmittel und endend mit dem Tacrolimus und dem Farbstoff, Mischen für 20 bis 30 Minuten, Überführen in tarierte Behälter und Dosieren in Fläschchen besteht.

## Revendications

1. Procédé de préparation d'une composition pharmaceutique pour suspension orale de tacrolimus ou d'un de ses sels, hydrates ou solvates pharmaceutiquement acceptables, ladite composition étant une poudre contenant du tacrolimus ou un de ses sels, hydrates ou solvates pharmaceutiquement acceptables, un colorant et un diluant ou facultativement d'autres excipients pharmaceutiques ; le procédé consistant à tamiser chacun des composants de la formulation, à les verser dans un mélangeur les uns après les autres, en commençant par le diluant et en laissant le tacrolimus et le colorant pour la fin, à mélanger pendant 20 à 30 minutes, à la placer dans des récipients tarés et à la doser en flacons.
